# EUROPÄISCHE PATENTSCHRIFT

(11) **EP 1 558 315 B1**
(45) Veröffentlichungstag und Bekanntmachung des Hinweises auf die Patenterteilung: **30.12.2009**
(21) Anmeldenummer: 03809748.1
(22) Anmeldetag: 30.10.2003
(51) Int. Cl.: A61M 15/00

(54) **INHALATIONSTHERAPIEVORRICHTUNG**
INHALATION THERAPY DEVICE
APPAREIL DE THERAPIE PAR INHALATION

(30) Priorität: 30.10.2002 DE 10250625
(43) Veröffentlichungstag der Anmeldung: 03.08.2005
(73) Patentinhaber: PARI Pharma GmbH, 82319 Starnberg (DE)
(72) Erfinder: FEINER, Franz, 81479 München (DE); BORGSCHULTE, Markus, 81543 München (DE); ACHTZEHNER, Wolfgang, 82239 Alling (DE); KUNSCHIR, Eduard, 80469 München (DE); LASS, Josef, 80798 München (DE)
(74) Vertreter: HOFFMANN EITLE
(86) Internationale Anmeldenummer: PCT/EP2003/012076
(87) Internationale Veröffentlichungsnummer: WO 2004/039442

(56) Entgegenhaltungen:
- EP-A- 1 005 917
- EP-A- 1 219 314
- EP-A- 1 304 130
- EP-A- 1 304 131
- WO-A-93/09881
- WO-A-03/057292
- DE-C- 19 953 317

## Beschreibung

Die Erfindung betrifft Inhalationstherapievorrichtungen mit einer schwingfähigen Membran zur Vernebelung einer Flüssigkeit.

Aus der Patentschrift DE 199 53 317 C1 ist ein Inhalationsvernebler der eingangs genannten Art bekannt. Der dort beschriebene Aerosolmembrangenerator umfasst einen zylindrischen Flüssigkeitsvorratsbehälter, der auf einer Stirnseite von einer Membran in Form einer Kreisscheibe begrenzt wird.

Eine in den Flüssigkeitsbehälter eingefüllte Flüssigkeit berührt die dem Flüssigkeitsvorratsbehälter zugewandte Seite der Membran. Ferner beschreibt DE 199 53 317 C1 einen Schwingungsgenerator, beispielsweise ein Piezo-Kristall, der die Membran kreisförmig umgibt und mit dieser so verbunden ist, dass die Membran mit Hilfe des Schwingungsgenerators und einer elektrischen Ansteuerschaltung in Schwingungen versetzt werden kann.

Durch die Öffnungen der in Schwingung versetzten Membran hindurch wird die auf der einen Seite an die Membran grenzende Flüssigkeit auf die andere Seite der Membran gefördert und an dieser Seite in die Mischkammer hinein als Aerosol abgegeben.

Aus dem Gebrauchsmuster DE 295 01 569 ist ein Ultraschall-Flüssigkeitszerstäuber mit einem Piezokristall bekannt, der von einer Oszillatorschaltung mit einer elektrischen Schwingung beaufschlagt wird, wobei die Oszillatorschaltung von einem Stromversorgungsgerät gespeist wird.

DE 295 01 569 beschreibt eine Oszillatorschaltung, welche eine Strombegrenzungsschaltung umfasst und mit einer elektronischen Temperaturbegrenzungsschaltung verbunden ist, die ein am Piezokristall auftretendes temperaturabhängiges elektrisches Signal in einer Schwellwertschaltung vergleicht, deren Vergleichssignal bei Erreichen einer Grenztemperatur im Piezokristall eine bistabile Schaltung ansteuert, die den Oszillator sperrend beaufschlagt.

Dabei ist die Offenbarung aus DE 295 01 569 auf einen Schutzmechanismus für einen Ultraschall-Flüssigkeitszerstäuber gerichtet, in welchem der Piezokristall selbst die Flüssigkeit in Schwingungen versetzt und in Kontakt mit einer vergleichsweise großen Flüssigkeitsmenge steht. Dazu muss der in der DE 295 01 569 beschriebene Flüssigkeitszerstäuber entsprechend große Ströme verwenden, um die große Flüssigkeitsmenge in Schwingung zu versetzen.

Durch diese großen Ströme und die dadurch auftretenden großen Temperaturunterschiede ist ein ständiger Kontakt des Piezokristalls mit Flüssigkeit notwendig, um einer Zerstörung des Piezokristalls vorzubeugen. Ist keine Flüssigkeit mehr vorhanden, erhitzt sich der Piezokristall sehr schnell und wird zerstört, wenn der Schwingkreis, der den Zerstäuber betreibt, nicht sofort abgeschaltet wird.

In Inhalationsverneblern der eingangs beschriebenen Art, also in Inhalationsverneblern mit Membranaerosolgenerator fließen nur sehr viel kleinere Ströme und es treten deswegen auch nur vergleichsweise kleine Temperaturunterschiede auf. Das Fehlen von Flüssigkeit führt in solchen Inhalationsverneblern nicht unmittelbar zu einer thermisch bedingten Beschädigung der piezo-elektrischen Elemente. Allerdings kann es bei Leerlauf eines Membraninhalationsverneblers in seltenen Fällen zu einem Bruch der Membran kommen.

Bei Inhalationsverneblern mit Membranaerosolgenerator besteht aber ebenfalls das Bedürfnis, das Vorhandensein von zu vernebelnder Flüssigkeit sicher zu erfassen. Denn zum einen wird dadurch die Grundlage für eine sehr hohe Dosisgenauigkeit geschaffen und zum anderen wird es möglich, dem Patienten das Ende einer Therapiesitzung zuverlässig anzuzeigen. Darüber hinaus kann durch die direkte Abschaltung der Inhalationstherapievorrichtung z.B. ein Akku geschont werden.

Dabei ist die Verwendung eines Schutzmechanismus, wie in DE 295 01 569 beschrieben, in Inhalationsverneblern der hier in Rede stehenden Art nicht notwendig und aufgrund der sehr viel kleineren Ströme und Temperaturveränderungen auch nicht möglich.

Ferner ist aus EP 1 219 314 A1 ein Vernebler mit einer Flüssigkeitsabgabevorrichtung mit einem Magnetventil bekannt, das die genau dosierte Zuführung von kleinen Flüssigkeitsmengen zu der Membran bewerkstelligt. Einzelne der Membran zugeführte Flüssigkeitsmengen werden vernebelt. Dabei ist eine Sensorelektrode vorgesehen, die in Verbindung mit der Membran als Gegenelektrode dazu dient, das Vorliegen eines Flüssigkeitstropfens an der Membran zu erfassen.

Aus WO 93/09881 ist ein Ultraschallvernebler mit einem Steuerungssystem bekannt, bei dem ein elektrisch zu betätigender Ultraschallübertrager in Form eines piezo-elektrischen Kristalls vorgesehen ist und der Übertrager eine Schwingung in einer zu vernebelnden Flüssigkeit hervorruft, wobei der Übertrager eine Strommessvorrichtung zur Messung des Übertragerantriebsstroms und eine Vergleichsvorrichtung zum Vergleichen dieser Messung mit einem vorbestimmten Schwellwert aufweist.

Aufgabe der vorliegenden Erfindung ist es demnach, eine Inhalationstherapievorrichtung mit Membran-Aerosolgenerator so auszugestalten, dass eine

Erhöhung der Dosisgenauigkeit erreichbar wird.

Diese Aufgabe wird durch eine Inhalationstherapievorrichtung mit den im Patentanspruch 1 angegebenen Merkmalen gelöst. Vorteilhafte Ausgestaltungen ergeben sich aus den Unteransprüchen.

Durch eine exakte Erfassung des Verbrauchs des eingefüllten Medikaments ermöglichst die Erfindung eine höhere Dosiergenauigkeit des Medikaments für den Patienten, erlaubt eine bessere Konzentration des Patienten auf die Therapie und erhöht die Benutzerfreundlichkeit der Inhalationstherapievorrichtung, nicht zuletzt durch eine längere Laufzeit im Akkubetrieb.

Durch die Möglichkeit der Feststellung, ob noch Flüssigkeit vorhanden ist, ist es möglich, die Inhalationstherapievorrichtung automatisch abzuschalten.

Die Erfindung wird nachstehend anhand von zeichnerisch dargestellten Ausführungsbeispielen näher erläutert, aus welchen weitere Vorteile und Merkmale der Erfindung hervorgehen. Es zeigt:
- Fig. 1: eine schematische Darstellung einer beispielhaften Inhalationsvorrichtung,
- Fig. 2: ein Flussdiagramm, das ein Verfahren zur Feststellung des Vorhandenseins von Flüssigkeit in der Inhalationsvorrichtung graphisch darstellt gemäss Fig. 1,
- Fig. 3: eine schematische Darstellung einer Inhalationsvorrichtung gemäß eines Ausführungsbeispiels,
- Fig. 4: ein Flussdiagramm, das ein Verfahren zur Feststellung des Vorhandenseins von Flüssigkeit in der Inhalationsvorrichtung graphisch darstellt gemäß eines Ausführungsbeispiels, und
- Fig. 5: einen beispielhaften zeitlichen Verlauf einer Messkurve eines erfassten elektrischen Parameters bei Verwendung von zwei unterschiedlichen Schwingungsfrequenzen für die Membran gemäß eines Ausführungsbeispiels.

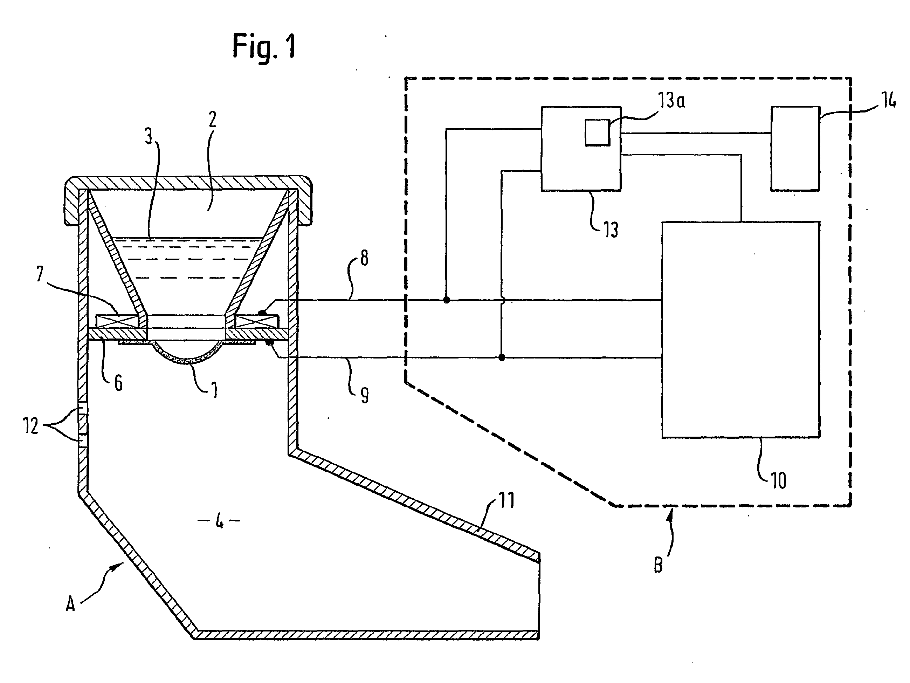

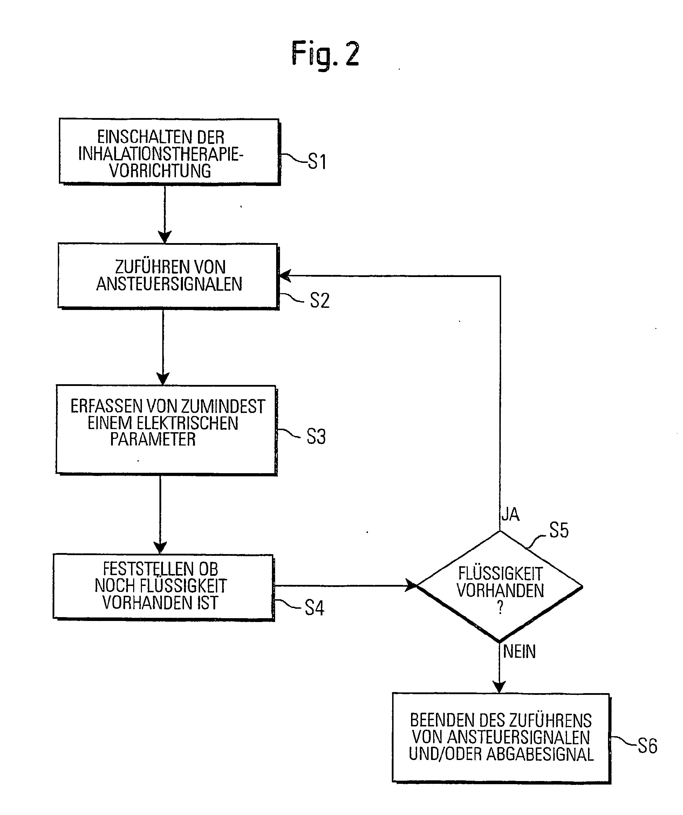

Zur Vorbereitung der Beschreibung eines Ausführungsbeispiels der Erfindung wird im Folgenden eine Inhalationsvorrichtung beispielhaft unter Bezugnahme auf die Fig. 1 und 2 näher erläutert.

Fig. 1 zeigt eine erfindungsgemäße Inhalationstherapievorrichtung, bei der in einer Verneblereinheit A mit Hilfe einer Membran (1) eine in einem Flüssigkeitsreservoir (2) bevorratete Flüssigkeit (3) in einen Vernebelungshohlraum (4) hinein vernebelt wird.

Die Vernebelung findet dann statt, wenn die Membran (1) in Schwingungen versetzt wird. Dazu ist die Membran (1) an einer Supporteinheit (6) befestigt, welche die Membran (1) trägt und an der auch eine elektro-mechanische Wandlereinheit (7), beispielsweise ein Piezoelement, befestigt ist.

Die Membran (1), die Supporteinheit (6) und die elektromechanische Wandlereinheit (7) sind bei dem hier beschriebenen Ausführungsbeispiel rotationssymmetrisch ausgebildet und bilden zusammen ein schwingungsfähiges Gebilde.

Über Anschlussleitungen (8,9) kann der elektro-mechanischen Wandlereinheit (7) ein Ansteuerungssignal von einer Steuereinrichtung (10) zugeführt werden, die bei dem hier beschriebenen Beispiel in einer separaten Steuerungseinheit B untergebracht ist. Wenn das Ansteuerungssignal zugeführt wird, wird das schwingfähige Gebilde (1, 6,7) in Schwingungen versetzt und die Flüssigkeit (3) durch die Membran (1) hindurch vernebelt.

Ein Patient kann das im Vernebelungshohlraum (4) bereitgestellte Aerosol am Mundstück (11) des Verneblers einatmen. Für die Zuführung einer ausreichenden Atemluftmenge sind im Gehäuse des Verneblers ein oder mehrere Atemöffnungen (12) vorgesehen, durch die Umgebungsluft in den Hohlraum (4) während des Einatmens eintreten und aus denen die Atemluft des Patienten aus dem Hohlraum (4) während des Ausatmens austreten kann.

Verschiedene elektrische Eigenschaften des schwingfähigen Gebildes (1, 6,7) (z. B. Strom, Spannung, Phasenverschiebung) sind insbesondere von der Kapazität der elektro-mechanischen Wandlereinheit (7) abhängig. Das schwingfähige Gebilde (1, 6, 7) zeigt bei Vernebelung und bei Betrieb ohne Flüssigkeit ganz bestimmte Charakteristika, die sich in den elektrischen Parametern des schwingfähigen Gebildes widerspiegeln. Die Betriebszustände mit und ohne Flüssigkeit an der Membran lassen sich zuverlässig anhand dieser elektrischen Parameter feststellen. Als elektrische Parameter eignen sich in besonderem Maße die Stromaufnahme (Strom), die Leistungsaufnahme (Leistung) und die Strom/Spannung-Phasenverschiebung (Phasenlage).

Um zumindest einen der elektrischen Parameter zu erfassen, ist erfindungsgemäß eine Erfassungseinrichtung (13) vorgesehen, die so ausgelegt und mit dem schwingfähigen Gebilde (1, 6, 7) und/oder der Steuereinrichtung (10) verbunden ist, dass der zumindest eine elektrische Parameter der Erfassungseinrichtung (13) zugeführt wird. Dazu sind beispielsweise die Anschlussleitungen (8, 9) so ausgelegt, dass während des Betriebs der Steuereinheit (10) mindestens ein elektrischer Parameter des schwingfähigen Gebildes (1, 6, 7) über die Anschlussleitungen (8, 9) zu der Erfassungseinrichtung (13) übermittelt wird und von dieser erfasst werden kann.

Aus der Erfassung von zumindest einem elektrischen Parameter des schwingfähigen Gebildes (1, 6, 7) (z.B. Abgriff der Spannung, der Stromaufnahme oder der Strom/Spannung-Phasenlage am Piezo-Kristall der Membran) aufgrund der Charakteristika des schwingfähigen Gebildes (1, 6, 7) kann auf den Betriebszustand zurückgeschlossen und dadurch erkannt werden, ob noch Flüssigkeit (3) im Flüssigkeitsreservoir (2) vorhanden ist.

Die Erfassung des mindestens einen elektrischen Parameters des schwingfähigen Gebildes (1, 6, 7) durch die Erfassungseinrichtung (13) kann kontinuierlich oder in diskreten zeitlichen Abständen erfolgen.

Die Feststellung des Betriebszustandes, also die Feststellung, ob Flüssigkeit vorhanden ist oder nicht, erfolgt in der Erfassungseinrichtung (13) vorzugsweise durch Vergleich des erfassten Wertes des zumindest einen Parameters mit einem Wert der für diesen Parameter in der Erfassungseinrichtung hinterlegt ist. Dafür umfasst die Erfassungseinrichtung (13) beispielsweise einen Speicher (13a).

Stellt die Erfassungseinrichtung (13) durch Vergleich eines erfassten Wertes mit einem hinterlegten Wert fest, dass keine Flüssigkeit (3) im Flüssigkeitsreservoir (2) mehr bevorratet ist, dann gibt die Erfassungseinrichtung (13) in einer bevorzugten Ausführungsform ein Signal an die Steuereinrichtung (10) ab, die wiederum automatisch die Zuführung von Ansteuersignalen zu dem schwingfähigen Gebilde (1, 6, 7) stoppt, d.h. die Inhalationstherapievorrichtung automatisch abschaltet.

In einer alternativen Ausgestaltung gibt die Erfassungseinrichtung (zusätzlich) ein optisches oder akustisches Signal ab, um dem Patienten anzuzeigen, dass das Inhalationstherapiegerät die bevorratete Flüssigkeit (3) im Flüssigkeitsreservoir (2) verbraucht hat, was dem Patienten das Ende der Therapiesitzung signalisiert. Der Patient kann dann seinerseits das Inhalationstherapiegerät ausschalten, sofern eine automatische Abschaltung nicht zusätzlich zu dem optischen/akustischen Signal vorgesehen ist.

Für die Abgabe des akustischen/optischen Signals umfasst die Inhalationstherapievorrichtung eine Signalgebereinrichtung (14), die mit der Erfassungseinrichtung (13) (oder alternativ der Steuereinrichtung) verbunden ist.

Das zu diesem Zweck abgegebene akustische Signal kann in einem kurzem Signalton von 0,5 bis 2 sec Dauer bestehen. Diese akustischen Signale sind aber nicht auf reine Töne beschränkt, sondern es können auch Tonfolgen oder aufgezeichnete oder synthetische Sprachsignale zum Einsatz kommen.

Fig. 2 zeigt ein Flussdiagramm, anhand dessen nunmehr ein möglicher Ablauf einer Therapiesitzung erläutert wird.

Durch das Einschalten der Inhalationstherapievorrichtung (Schritt S1) werden Ansteuerungssignale dem schwingfähigen Gebilde zugeführt (Schritt S2). Unmittelbar anschließend überprüft die Erfassungseinrichtung (13), ob die Anfangsbedingungen für eine Therapiesitzung gegeben sind, d.h. sie stellt fest, ob Flüssigkeit (3) im Flüssigkeitsreservoir (2) vorhanden ist.

Im Einzelnen erfasst die Erfassungseinrichtung (13) zumindest einen elektrischen Parameter des schwingfähigen Gebildes (1, 6, 7) (Schritt S3) und stellt auf der Basis des erfassten Wertes des zumindest einen elektrischen Parameters fest, ob Flüssigkeit vorhanden ist oder nicht (Schritt S4).

Dazu greift die Erfassungseinrichtung (13) beispielsweise auf empirisch ermittelte Werte für den erfassten elektrischen Parameter zurück, die in der Erfassungseinrichtung auf geeignete Weise hinterlegt, beispielsweise in dem Halbleiterspeicher (13a) gespeichert sind, der in Figur 1 gezeigt ist oder verwendet einen Wert des zumindest einen Parameters, der in einem vorherigen Durchlauf der Schleife (s.u.) erfasst wurde. Dieser Wert wird für diesen Zweck von der Erfassungseinrichtung (13) in geeigneter Form abgelegt, beispielsweise in dem Halbleiterspeicher (13a) gespeichert.

Wird durch Wertevergleich das Vorhandensein von Flüssigkeit festgestellt (Schritt S5), wird die Zuführung des Ansteuerungssignals zu dem schwingfähigen Gebilde (1, 6, 7) fortgesetzt; der Steuerungsablauf kehrt zum Schritt S2 zurück.

Wird hingegen in Schritt S5 festgestellt, dass keine Flüssigkeit vorhanden ist, wird die Zuführung des Ansteuerungssignals zu dem schwingfähigen Gebilde (1, 6, 7) sofort wieder beendet (Schritt S6). Zusätzlich oder alternativ kann ein optisches / akustisches Signal abgegeben werden (Schritt S6).

Die Schleife der Schritte S2 bis S5 wird kontinuierlich oder in regelmäßigen Abständen (diskrete Zeitschritte) durchlaufen, um während der Therapiesitzung das Vorhandensein von Flüssigkeit zu überprüfen und um ggf. die Zuführung des Ansteuerungssignals zu dem schwingfähigen Gebilde und damit die Vernebelung zu beenden.

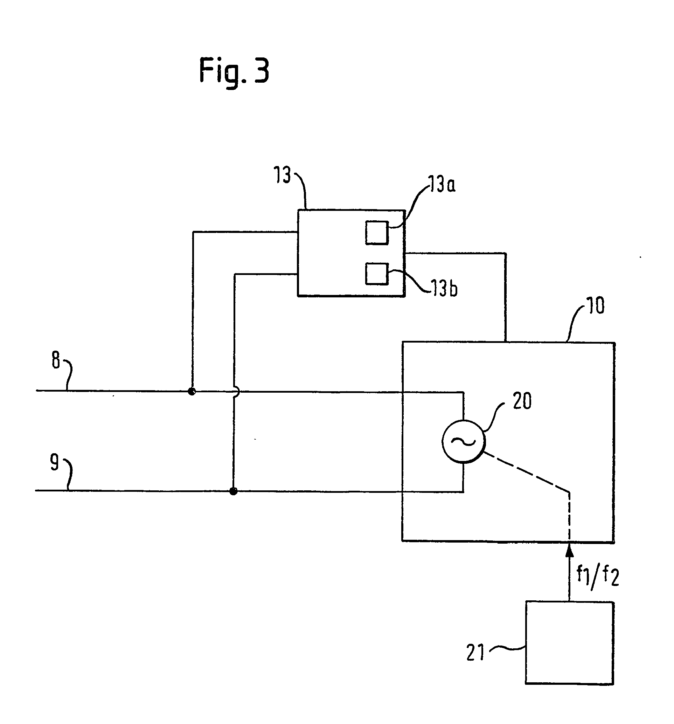

Als nächstes wird ein Ausführungsbeispiel für die Erfindung anhand der Figuren 3 bis 5 erläutert, wobei die vorangegangene Beschreibung einer Inhalationsvorrichtung grundlegend einbezogen, aber im Folgenden nicht wiederholt wird.

Fig. 3 zeigt ein Ausführungsbeispiel einer Inhalationstherapievorrichtung, bei der erfindungsgemäß mindestens zwei unterschiedliche Schwingungsfrequenzen für die Membran erzeugt und der Membran alternierend zugeführt werden. Bei der ersten Frequenz f1 handelt es sich um die Ansteuerfrequenz, die dem schwingungsfähigen Gebilde (1,6, 7) zugeführt wird, um die Membran in Schwingungen zu versetzen und die Flüssigkeit zu vernebeln. Bei der zweiten Frequenz f2 handelt es sich demgegenüber um eine Frequenz zur Bestimmung des Betriebszustandes des schwingungsfähigen Gebildes (1,6, 7). Typischerweise sind die Zeitspannen, in denen die zweite Frequenz f2 dem schwingungsfähigen Gebilde (1,6, 7) zugeführt wird, sehr viel kürzer als die Zeiträume, in denen die erste Frequenz f1 zugeführt wird. Denn die Zuführung der zweiten Frequenz f2 dient Messzwecken und darf die Erzeugung des Aerosols nur in geringstem Masse stören.

Wie in Fig. 3 gezeigt, umfasst die Steuereinheit (10) in dieser erfindungsgemäßen Ausführungsform dazu zum Beispiel einen Oszillator. (20), der mindestens zwei unterschiedliche Schwingungsfrequenzen (f1, f2) für die Membran (1) erzeugen kann.

Eine Umschalteinrichtung (21) schaltet den Oszillator (20) der Steuereinheit (10) zu vorgegebenen Zeitpunkten zwischen der normalen Betriebsfrequenz f1 und der Messfrequenz f2 um, wobei in Intervallen, in denen die normale Betriebsfrequenz f2 verwendet wird, die Inhalationstherapievorrichtung die vorhandene Flüssigkeit vernebelt.

Die Erfassungseinheit (13) speichert die erfassten Werte des mindestens einen elektrischen Parameters, die während der Verwendung der Messfrequenz 2 erfasst wurden, um diese Messwerte auch über einen längeren Zeitraum auswerten zu können.

Die Feststellung des Betriebszustandes, also die Feststellung, ob Flüssigkeit vorhanden ist oder nicht, erfolgt in der Erfassungseinrichtung (13) dann entweder durch Vergleich eines Wertes des zumindest einen Parameters, der während der normalen Betriebsfrequenz f1 erfasst wurde, mit einem Wert, der für diesen Parameter in der Erfassungseinrichtung hinterlegt ist (dafür umfasst die Erfassungseinrichtung (13) beispielsweise einen Speicher (13a)) oder durch Auswertung von Werten, die während der Verwendung der Messfrequenz f2 von einem elektrischen Parameter aufgezeichnet wurden. Der Betriebszustand kann natürlich auch durch Verwendung von Werten beider erfasster Parametersätze festgestellt werden.

Ferner ist es auch möglich, die erfassten Werte des elektrischen Parameters während der normalen Betriebsfrequenz f1 in der Erfassungseinrichtung (13) aufzuzeichnen (beispielsweise in einem Speicher (13b)), um auch diese über einen längeren Zeitraum auswerten zu können.

Stellt die Erfassungseinrichtung (13) fest, dass keine Flüssigkeit (3) im Flüssigkeitsreservoir (2) mehr bevorratet ist, dann gibt die Erfassungseinrichtung (13) in einer bevorzugten Ausführungsform ein Signal an die Steuereinrichtung (10) ab, die wiederum automatisch die Zuführung von Ansteuersignalen zu dem schwingfähigen Gebilde (1, 6,7) stoppt, d. h. die Inhalationstherapievorrichtung automatisch abschaltet.

Im Übrigen wird auf die Beschreibung der Inhalationsvorrichtung gemäß Figur 1 und den Betrieb gemäß Figur 2 verwiesen.

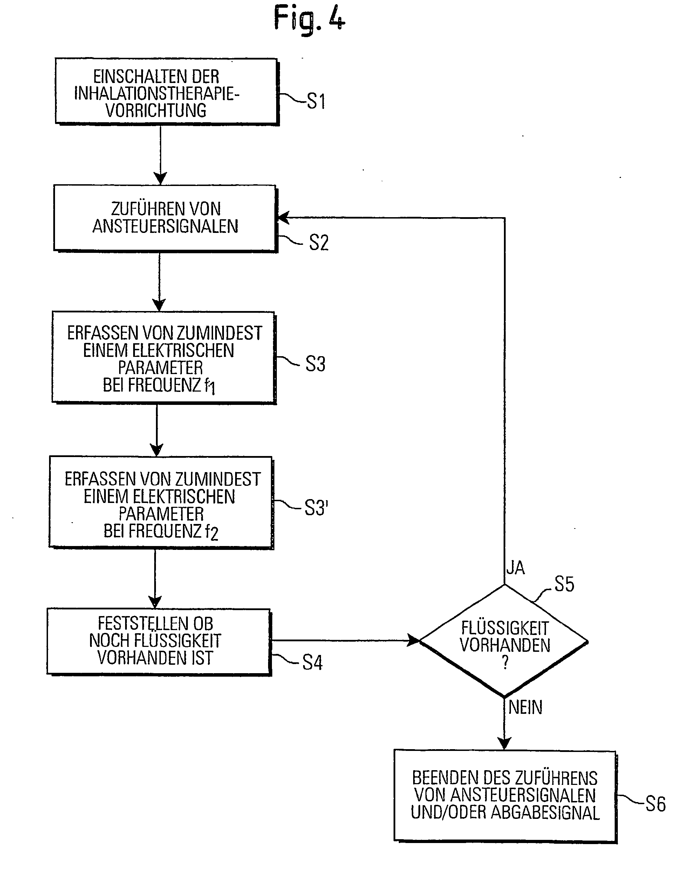

Fig. 4 zeigt ein Flussdiagramm, anhand dessen nunmehr ein möglicher Ablauf einer Therapiesitzung gemäß dem erfindungsgemäßen Ausführungsbeispiel erläutert wird.

Durch das Einschalten der Inhalationstherapievorrichtung (Schritt S1) werden Ansteuerungssignale mit normaler Betriebsfrequenz fl dem schwingfähigen Gebilde zugeführt (Schritt S2). Unmittelbar anschließend überprüft die Erfassungseinrichtung (13), ob die Anfangsbedingungen für eine Therapiesitzung gegeben sind, d.h. sie stellt fest, ob Flüssigkeit (3) im Flüssigkeitsreservoir (2) vorhanden ist.

Im Einzelnen erfasst die Erfassungseinrichtung (13) zumindest einen elektrischen Parameter des schwingfähigen Gebildes (1, 6, 7) bei Verwendung der normalen Betriebsfrequenz f₁ (Schritt S3) und stellt auf der Basis des erfassten Wertes des zumindest einen elektrischen Parameters fest, ob Flüssigkeit vorhanden ist oder nicht (Schritt S4).

Dazu greift die Erfassungseinrichtung (13) beispielsweise auf empirisch ermittelte Werte für den erfassten elektrischen Parameter zurück, die in der Erfassungseinrichtung auf geeignete Weise hinterlegt, beispielsweise in dem Halbleiterspeicher (13a) gespeichert sind, der in Figur 3 gezeigt ist.

Wird das Vorhandensein von Flüssigkeit festgestellt (Schritt S5), wird die Zuführung des Ansteuerungssignals zu dem schwingfähigen Gebilde (1, 6, 7) fortgesetzt; der Steuerungsablauf kehrt zum Schritt S2 zurück.

Wird hingegen in Schritt S5 festgestellt, dass keine Flüssigkeit vorhanden ist, wird die Zuführung des Ansteuerungssignals zu dem schwingfähigen Gebilde (1, 6, 7) sofort wieder beendet (Schritt S6). Zusätzlich oder alternaiv kann ein optisches / akustisches Signal abgegeben werden (Schritt S6).

Nach dem Initialisierungsschritt wird die Schleife der Schritte S2 bis S5 kontinuierlich oder in regelmäßigen Abständen (diskrete Zeitschritte) durchlaufen, um während der Therapiesitzung das Vorhandensein von Flüssigkeit zu überprüfen und um ggf. die Zuführung des Ansteuerungssignals zu dem schwingfähigen Gebilde und damit die Vernebelung zu beenden. Dabei wird in vorgegebenen Abständen zwischen der normalen Betriebsfrequenz f1 und der Messfrequenz f2 umgeschaltet. Die Längen der Zeitintervalle, während derer die Messfrequenz f₂ verwendet wird, sind dabei so gewählt, dass der Vernebelungsbetrieb nicht gestört wird. Typischerweise sind die Zeitintervalle der Messfrequenz mindestens um den Faktor 10 kleiner.

Im Einzelnen erfasst die Erfassungseinrichtung (13) zumindest einen elektrischen Parameter des schwingfähigen Gebildes (1, 6, 7) bei der normalen Betriebsfrequenz f₁ (Schritt S3) oder der Messfrequenz f₂ (Schritt 3') und stellt auf der Basis der erfassten Werte des zumindest einen elektrischen Parameters fest, ob Flüssigkeit vorhanden ist oder nicht (Schritt S4).

Dazu greift die Erfassungseinrichtung (13) für die Werte, die mit der normalen Betriebsfrequenz f1 ermittelt wurden (Schritt 3) entweder auf empirisch ermittelte Werte für den erfassten elektrischen Parameter zurück, die in der Erfassungseinrichtung auf geeignete Weise hinterlegt, beispielsweise in dem Halbleiterspeicher (13a) gespeichert sind, der in Figur 3 gezeigt ist, oder verwendet einen Wert des zumindest einen Parameters, der in einem vorherigen Durchlauf der Schleife erfasst wurde. Dieser Wert wurde für diesen Zweck von der Erfassungseinrichtung (13) in geeigneter Form abgelegt, beispielsweise in dem Halbleiterspeicher (13a) gespeichert.

Die erfassten Werte des zumindest einen elektrischen Parameters des schwingfähigen Gebildes (1, 6, 7), die während der Verwendung der Messfrequenz f₂ ermittelt und im Speicher (13b) abspeichert wurden (Schritt 3') wertet die Erfassungseinrichtung (13) entweder wie die anderen Messwerte, oder vorzugsweise über einen längeren Zeitraum aus (Schritt 4).

Die Entscheidung, ob noch Flüssigkeit vorhanden ist, kann in diesem Ausführungsbeispiel auf beiden Arten von erfassten Werten des elektrischen Parameters beruhen. Dies erhöht die Sicherheit der Richtigkeit der Feststellung, ob noch Flüssigkeit vorhanden ist. Durch Betrachtung des Verlaufs der Messkurve über einen längeren Zeitraum, kann darüber hinaus die Zuverlässigkeit der Feststellung, ob Flüssigkeit vorhanden ist, noch weiter erhöht werden.

Die Erfindung ist aber nicht auf die Verwendung von zwei Frequenzen beschränkt. Es können mehrere Frequenzen für die beschriebene Vorrichtung verwendet werden.

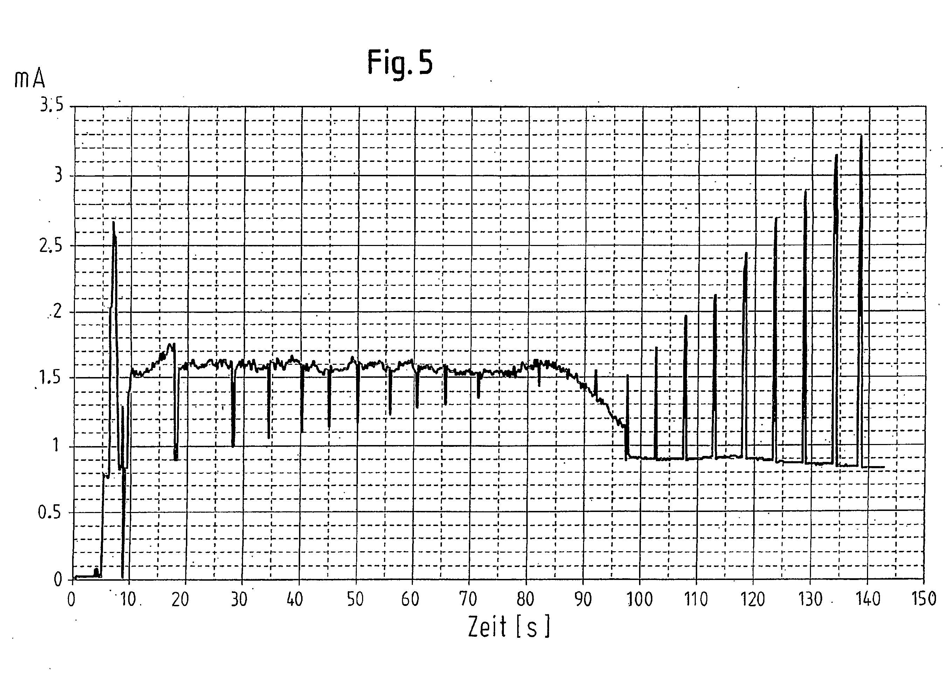

Fig. 5 zeigt einen beispielhaften zeitlichen Verlauf eines der erfassten elektrischen Parameter für den Fall, dass zwei unterschiedliche Frequenzen für die Membranschwingungen verwendet werden.

In Fig. 5 ist eine beispielhafte Messkurve dargestellt, die den Verlauf der erfassten Werte des zumindest einen elektrischen Parameters des schwingfähigen Gebildes (1, 6, 7) des erfindungsgemäßen Ausführungsbeispiels darstellt. Bei dem in der beispielhaften Messkurve dargestellten Messwert handelt es sich um die Stromaufnahme des schwingungsfähigen Gebildes (1,6, 7) in mA.

Der Verlauf im Zeitbereich von 0 bis ca. 17 sec ist auf den Einschaltvorgang zurückzuführen und kann vernachlässigt werden.

Über den Gesamtbereich der Messkurve ist erkennbar, dass in den Zeitintervallen, in denen ein Ansteuerungssignal mit der Betriebsfrequenz fl an das schwingungsfähige Gebilde angelegt wird, sich zunächst ein Wert von ca. 1,6 mA einstellt, der zwischen der 80. und 97. Sekunde auf einen Wert von 0,9 mA abfällt. Dieser Verlauf der Messkurve entspricht auch dem grundsätzlichen Verlauf der erfassten Werte bei einer Ausführungsform der Erfindung, bei der nur die Betriebsfrequenz fl eingesetzt wird.

In Fig. 5 sind daneben die kurzen Zeitintervalle erkennbar, in denen ein Ansteuersignal mit der Messfrequenz f2 an das schwingungsfähige Gebilde (1,6, 7) angelegt wird. Diese Zeitintervalle entsprechen den in Fig. 5 erkennbaren Spitzen, wobei auch deutlich wird, dass diese Zeitintervalle kürzer sind als die zwischen den Spitzen liegenden Zeitintervalle, in denen die Betriebsfrequenz fl vorliegt.

Im Zeitintervall von der 15. bis 85. Sekunde liegen die Messwerte, die bei vorliegender Betriebsfrequenz fl erfasst werden, in einem sehr engen Bereich bei ca. 1,6 mA. Ab der 85ten Sekunde fällt die Messkurve der Werte bei der Betriebsfrequenz fl bis auf etwa 0,9 mA ab. Ab der ca. 97ten Sekunde sind die Messwerte wieder im Wesentlichen konstant.

In den sehr kurzen Zeitintervallen, in denen die Messfrequenz 2 vorliegt, sind die Spitzenwerte interessant, die über den gesamten Kurvenverlauf hin ansteigen. Im Zeitintervall von der 15. bis zur 95. Sekunde verlaufe die. Spitzenwerte entlang einer Geraden mit einer ersten Steigung ; im Zeitraum ab der 95. Sekunde verlaufen die Spitzenwerte der Messwerte bei der Messfrequenz 2 entlang einer Geraden mit einer zweiten Steigung, die grösser ist als die erste Steigung. Dieser Wechsel in der Steigung ist ein deutlich erfassbares Anzeichen für das Fehlen von Flüssigkeit an der Membran bzw. an dem schwingungsfähigen Gebilde (1,6, 7) der Inhalationstherapievorrichtung.

Somit bietet das Ausführungsbeispiel der erfindungsgemäßen Inhalationstherapievorrichtung zwei Messkurvenverläufe, mit deren Hilfe das Fehlen von Flüssigkeit festgestellt werden kann. Denn einerseits fällt die Messkurve der Werte bei der Betriebsfrequenz fl ab, wenn die Flüssigkeit verbraucht ist und andererseits wechselt die Zunahmerate der Spitzenwerte der bei der Messfrequenz 2 ermittelten Werte.

Die in Fig. 5 gezeigte Messkurve ist beispielhaft und kann sich bei unterschiedlichen Auslegungen des Inhalationstherapiegerätes verändern. Insbesondere die angegebenen Werte und Zeiträume können sich in Abhängigkeit von der konkreten Ausgestaltung der Vorrichtung unterscheiden.

## Patentansprüche

1. Inhalationstherapievorrichtung mit
einer schwingungsfähigen Membran (1) für die Vernebelung einer Flüssigkeit (3),
einem Flüssigkeitsreservoir (2) für die Bevorratung der zu vernebelnden Flüssigkeit (3) derart, dass eine in dem Flüssigkeitsreservoir bevorratete Flüssigkeit die dem Flüssigkeitsreservoir zugewandte Seite der Membran (1) berührt,
einer Schwingungserzeugungseinrichtung (6, 7), die zumindest eine Anschlusseinrichtung (8, 9) für die Zuführung eines Ansteuersignals aufweist und durch die die Membran (1) in Schwingungen versetzt wird, wenn das Ansteuersignal zugeführt wird, so dass eine auf einer Seite der Membran anstehende Flüssigkeit durch die Membran hindurch vernebelt wird und auf der anderen Seite der Membran als Aerosol vorliegt, und
einer Steuereinrichtung (10), von der ein Ansteuersignal der zumindest einen Anschlusseinrichtung (8, 9) der Schwingungserzeugungseinrichtung (6, 7) zuführbar ist, so dass die Schwingungserzeugungseinrichtung (6, 7) die Membran (1) in Schwingungen versetzt,
**dadurch gekennzeichnet, dass**
die Steuereinrichtung (10) alternierend Ansteuersignale mit mindestens zwei unterschiedlichen Frequenzen (f₁,f₂) erzeugt und
eine Erfassungseinrichtung (13) vorgesehen ist,
- die zumindest einen elektrischen Parameter des die Membran (1) und die Schwingungserzeugungseinrichtung (6, 7) umfassenden schwingfähigen Gebildes erfasst,
- die auf der Basis von erfassten Werten des zumindest einen Parameters bei den mindestens zwei unterschiedlichen Frequenzen (f₁,f₂) das Vorhandensein von zu vernebelnder Flüssigkeit feststellt,
wobei es sich bei dem zumindest einen elektrischen Parameter um die Stromaufnahme, die Leistungsaufnahme oder die Strom/Spannung-Phasenverschiebung des Ansteuersignals handelt.

2. Inhalationstherapievorrichtung nach Anspruch 1, **dadurch gekennzeichnet, dass** die Zeitintervalle, in denen ein erstes Ansteuersignal mit einer ersten Frequenz (f₁) erzeugt wird, länger sind und vorzugsweise um mindestens den Faktor 10 länger sind als die Zeitintervalle, in denen ein Ansteuersignal mit einer zweiten Frequenz (f₂) erzeugt wird.

3. Inhalationstherapievorrichtung nach einem der Ansprüche 1 oder 2, **dadurch gekennzeichnet, dass** die erfassten Werte für eine Auswertung über einen längeren Zeitraum hinweg abgespeichert werden.

4. Inhalationstherapievorrichtung, nach einem der Ansprüche 1 bis 3, **dadurch gekennzeichnet, dass**
wenn die Erfassungseinrichtung (13) feststellt, dass keine Flüssigkeit (3) vorhanden ist,
die Erfassungseinrichtung (13)
die Zuführung von Ansteuersignalen durch die Steuereinrichtung (10) zu der Schwingungserzeugungseinrichtung (6,7) verhindert, und/oder
- die Erzeugung eines optischen und/oder akustischen Signals durch eine Signalgebereinrichtung (14) auslöst, um anzuzeigen, dass keine Flüssigkeit (3) vorhanden ist.

5. Inhalationstherapievorrichtung nach Anspruch 4,
**dadurch gekennzeichnet, dass**
das abgegebene akustische Signal aus einem kurzen Signalton besteht und/oder aus einer Tonfolge und/oder aus aufgezeichneten oder synthetischen Sprachsignalen.

6. Inhalationstherapievorrichtung nach einem der vorangegangenen Ansprüche, **dadurch gekennzeichnet, dass**
die Schwingungserzeugungseinrichtung (6, 7) eine elektromechanische Wandlereinheit (7), insbesondere ein piezoelektrisches Element, umfasst.

7. Inhalationstherapievorrichtung nach Anspruch 6,
**dadurch gekennzeichnet, dass**
die Schwingungserzeugungseinrichtung (6, 7) eine Supporteinheit (6) umfasst, an der die elektro-mechanische Wandlereinheit (7) und die Membran (1) befestigt sind.

8. Inhalationstherapievorrichtung nach einem der vorangegangenen Ansprüche, **dadurch gekennzeichnet, dass**
in der Steuereinrichtung (10) eine Energieversorgungseinheit für die Inhalationsvorrichtung integriert ist.

## Claims

1. Inhalation therapy device having
an oscillatable membrane (1) for the nebulisation of a liquid (3),
a liquid reservoir (2) for storage of the liquid (3) to be nebulised such that a liquid stored in the liquid reservoir contacts the side of the membrane (1) facing towards the liquid reservoir,
an oscillation generator (6, 7) which has at least one connection device (8, 9) for the delivery of an activating signal and by which the membrane (1) is set in oscillation when the activating signal is delivered, so that a liquid present on one side of the membrane is nebulised through the membrane and is in the form of an aerosol on the other side of the membrane, and
a control device (10) by which an activating signal can be delivered to the at least one connection device (8, 9) of the oscillation generator (6, 7), so that the oscillation generator (6, 7) sets the membrane (1) in oscillation,
**characterised in that**
the control device (10) alternately generates activating signals with at least two different frequencies (f₁, f₂) and
a detection device (13) is provided
- which detects at least one electrical parameter of the oscillatable structure encompassing the membrane (1) and the oscillation generator (6, 7),
- which detects the presence of liquid to be nebulised on the basis of detected values of the at least one parameter at the at least two different frequencies (f₁, f₂), the at least one electrical parameter being the current consumption, the power consumption or the current/voltage phase shift of the activating signal.

2. Inhalation therapy device according to claim 1,
**characterised in that** the intervals of time in which a first activating signal having a first frequency (f₁ is generated are longer, and preferably longer by a factor of at least 10, than the intervals of time in which an activating signal having a second frequency (f₂) is generated.

3. Inhalation therapy device according to either of claims 1 or 2, **characterised in that** the detected values are stored for evaluation over a longer period of time.

4. Inhalation therapy device according to any of claims 1 to 3, **characterised in that**
when the detection device (13) detects that no liquid (3) is present,
the detection device (13)
- prevents the delivery of activating signals by the control device (10) to the oscillation generator (6, 7), and/or
- triggers the generation of an optical and/or acoustic signal by a signal transmitter device (14) in order to indicate that no liquid (3) is present.

5. Inhalation therapy device according to claim 4,
**characterised in that**
the acoustic signal emitted consists of a short signal tone and/or a tone sequence and/or recorded or synthetic voice signals.

6. Inhalation therapy device according to any of the preceding claims, **characterised in that**
the oscillation generator (6, 7) comprises an electromechanical transducer unit (7), in particular a piezoelectric element.

7. Inhalation therapy device according to claim 6,
**characterised in that**
the oscillation generator (6, 7) comprises a support unit (6) to which are attached the electromechanical transducer unit (7) and the membrane (1).

8. Inhalation therapy device according to any of the preceding claims, **characterised in that**
an energy supply unit for the inhalation device is integrated in the control device (10).

## Revendications

1. Appareil de thérapie par inhalation, comprenant :
une membrane (1) susceptible de vibrer, pour la nébulisation d'un liquide (3),
un réservoir à liquide (2) pour le stockage du liquide (3) à nébuliser, de manière qu'un liquide stocké dans le réservoir à liquide, entre en contact avec la face, tournée vers le réservoir à liquide, de la membrane (1),
un dispositif générateur de vibrations (6, 7), présentant au moins un dispositif de raccordement (8, 9) pour l'amenée d'un signal de commande et au moyen duquel la membrane (1) est mise en vibrations, lorsque le signal de commande est amené, de manière qu'un liquide, présent sur une face de la membrane, soit nébulisé par la membrane et présent, sous forme d'aérosol, sur l'autre face de la membrane, et
un dispositif de commande (10), dont un signal de commande est susceptible d'être amené au au moins un dispositif de raccordement (8, 9) du dispositif générateur de vibrations (6, 7), de manière que le dispositif générateur de vibrations (6, 7) mette la membrane (1) en vibration,
**caractérisé en ce que**
le dispositif de commande (10) génère en alternance des signaux de commande ayant au moins deux fréquences (f₁, f₂) différentes, et
étant prévu un dispositif de détection (13),
- détectant au moins un paramètre électrique de la structure apte à vibrer, comprenant la membrane (1) et le dispositif générateur de vibrations (6, 7),
- constatant la présence de liquide à nébuliser, à partir de valeurs détectées du au moins un paramètre, pour les au moins deux fréquences (f₁, f₂) différentes, sachant que, concernant le au moins un paramètre électrique, il s'agit de la consommation de courant, de la consommation de puissance, ou du déphasage courant/tension du signal de commande.

2. Appareil de thérapie par inhalation selon la revendication 1, **caractérisé en ce que** les intervalles de temps, auxquels un premier signal de commande, d'une première fréquence (f₁) est généré, sont plus longs et, de préférence, plus longs d'au moins le facteur 10 que les intervalles de temps auxquels un signal de commande, d'une deuxième fréquence (f₂), est généré.

3. Appareil de thérapie par inhalation selon l'une des revendications 1 ou 2, **caractérisé en ce que** les valeurs détectées sont mémorisées pour une évaluation sur une longue période de temps.

4. Appareil de thérapie par inhalation selon l'une des revendications 1 à 3, **caractérisé en ce que,**
lorsque le dispositif de détection (3) constate qu'aucun liquide n'est présent,
le dispositif de détection (13)
- empêche l'amenée de signaux de commande, au moyen du dispositif de commande (10), au dispositif générateur de vibrations (6, 7), et/ou
- déclenche, au moyen d'un dispositif fournisseur de signaux (14), la génération d'un signal optique et/ou acoustique, pour afficher qu'aucun liquide (3) n'est présent.

5. Appareil de thérapie par inhalation selon la revendication 4, **caractérisé en ce que**
le signal acoustique émis est composé d'un bref son de signalisation et/ou d'une succession de sons et/ou de signaux vocaux, enregistrés ou synthétiques.

6. Appareil de thérapie par inhalation selon l'une des revendications précédentes, **caractérisé en ce que**
le dispositif générateur de vibrations (6, 7) comprend une unité convertisseuse (7) électromécanique, en particulier un élément piézoélectrique.

7. Appareil de thérapie par inhalation selon la revendication 6, **caractérisé en ce que**
le dispositif générateur de vibrations (6, 7) comprend une unité support (6), à laquelle l'unité convertisseuse (7) électromécanique et la membrane (1) sont fixées.

8. Appareil de thérapie par inhalation selon l'une des revendications précédentes, **caractérisé en ce qu'**
une unité d'alimentation en énergie pour l'appareil de thérapie par inhalation est intégrée dans le dispositif de commande (10).
